# EUROPEAN PATENT SPECIFICATION

(11) **EP 3 821 927 B1**
(45) Date of publication and mention of the grant of the patent: **19.01.2022**
(21) Application number: 19209288.0
(22) Date of filing: 14.11.2019
(51) Int. Cl.: A61K 31/045, A61K 31/164, A61K 31/4174, A61K 31/455, A61K 31/505, A61K 36/28, A61K 36/886, A61M 15/08, A61M 35/00, A61K 9/06, A61K 9/08, A61K 47/44, A61M 11/00, B05B 11/00, A61M 15/00

(54) **DEVICE FOR INTERNAL AND EXTERNAL NASAL APPLICATION**
VORRICHTUNG ZUR INNEREN UND ÄUSSEREN NASALEN ANWENDUNG
DISPOSITIF D'APPLICATION NASALE INTERNE ET EXTERNE

(43) Date of publication of application: 19.05.2021
(73) Proprietor: GSK Consumer Healthcare SARL, 1260 Nyon (CH)
(72) Inventor: ATHANASE, Thierry, 1197 Prangins (CH); BANERJEE, Sriman, Warren, New Jersey 07059 (US); SABHERWAL, Amit, Gurugram, Haryana 12202 (IN)
(74) Representative: Gundel, Isabelle

(56) References cited:
- WO-A1-2019/006173
- WO-A2-2019/125330

## Description

This invention relates to a novel combination product. The combination product comprises a nasal spray product comprising a dispensing head mounted on a top of a container containing a liquid formulation for nasal administration.

Intranasal administration of a liquid formulation, for example a drug formulation or a medicinal product formulation frequently employs a primary packaging for such formulation that comprises a container and a dispensing mechanism with a nozzle that is suitable to be introduced to a user's nostril and from which such liquid formulation is dispensed into the nostril. In particular for liquid formulations containing an active ingredient, where it is important that the desired dose of the ingredient is administered, metered dose spray pumps are commonly used as a dispensing mechanism.

Metered dose spray pumps are for example known from EP1768789B3, EP1954403B1, or WO2017191205A1. Other relevant prior art documents are WO 2019/006173 A1 and WO 2019/125330 A2.

The nasal administration of saline solutions which may in addition contain active ingredients is a widely used method of treatment. Active ingredients which come into consideration are, for example, vasoconstrictors, such as xylometazoline, or antiallergic agents, such as cromoglycic acid or H1 receptor antagonists, e.g. dimethindene maleate. Another group of possible active ingredients is e.g. corticosteroids, such as beclomethasone or fluticasone.

The indications in which a certain nasally administered drug is to be applied are known in the art. For example, vasoconstrictors are e.g. used as nasal decongestants for alleviating the typical symptoms of common cold or flu, like running nose, obstructed nose etc., or in rhinitis or sinusitis. Antiallergic agents and corticosteroids are e.g. used in antiallergic conditions, e.g. hay fever, or in antiasthmatic or anti-inflammatory conditions.

Patients who suffer from any of these conditions do in general frequently use tissues to blow their nose or to clean their nose. This frequently leads to skin irritation and redness around the nose, which generally occurs soon after the onset of the symptoms which are treated by the intranasal administration of a liquid formulation. This irritation or redness can in certain circumstances and depending on the active ingredients even be exacerbated by the liquid formulation which has been administered, but does not stay in the nostrils.

Today, patients and consumers either leave this skin irritation untreated, or treat it with skin creams or balms sold for different purposes, and/or conceal it with make-up or other cosmetic products. However, existing cream or balm formulations in the market are not necessarily suitable for treating the irritation and redness around the nose, and the use of concealer or make-up does not treat the condition, and might even worsen it. Furthermore, using a nasal spray and a separate product such as a cream or balm is not convenient, especially when both products need to be used several times per day, and users are not staying at home but travel or commute to their workplace, school or other locations.

There is thus a need for a solution allowing users to conveniently treat the skin irritation and redness around the nose occurring frequently together with nasal congestion or a running nose.

This is achieved according to the invention by a novel combination product comprising a dispensing head mounted on a top of a container containing a liquid formulation for nasal administration, and a solidified skin-care balm for application around the nose contained in a balm holder element attached to a bottom of the container.

According to a preferred embodiment of the invention, the dispensing head is a metered dose spray pump.

According to a preferred embodiment of the invention, the balm holder element comprises a balm cover with a cylindrical portion surrounding the balm and a cover portion, and a base element on which the balm is mounted. According to a further preferred embodiment, the balm holder element is made from polypropylene (PP).

Typically, and as known in the art, the container containing the liquid formulation is made from high density polyethylene (HDPE).

Preferably the cylindrical portion of the balm cover is aligned with a cylindrical wall of the container containing the liquid formulation.

According to one preferred embodiment of the invention, the balm cover is attached to the bottom of the container. The user can then take off the balm holder element with the balm and apply the balm without having to handle the container and the dispensing head during application.

Preferably, the balm cover is glued to the bottom of the container. A suitable glue for attaching the balm cover to the container, in particular when the balm cover is made from PP and the container out of HDPE, is cyano-acrylate glue which is used together with a primer. No heating is necessary to create the bond, and such a glue is thus particularly suitable to be used with a pre-filled container, where the application of heat in an assembly process could compromise the stability of the liquid formulation contained in the container.

Preferably, the cover portion of the balm cover is dome-shaped and matches a recess in the bottom of the container containing the liquid formulation. This improves the bond between the balm cover and the container.

As an alternative to the glued connection the balm cover is clipped or snap-fitted to the bottom of the container. Depending on the design of the connection, this can also allow for an assembly or disassembly by the consumer.

According to an alternative embodiment, instead of the balm cover, the base element is attached to the bottom of the container. Similarly to what was described above, the base element can be glued, clipped, or snap-fitted to the container, or attached in any other suitable way, either removable or non-removable for the consumer.

It should be noted that the term "attached to the bottom" should not be understood as limited to an attachment of the balm cover or the base element to a bottom surface of the container, but that the balm holder element can also be attached to a bottom part of the cylindrical wall of the container.

According to a preferred embodiment of the invention, the liquid formulation contains a saline solution and an active ingredient selected from the group of vasoconstrictors, antiallergic agents and corticosteroids.

Active substances suitable for nasal administration are e.g. vasoconstrictors, e.g. xylometazoline, e.g. xylometazoline hydrochloride; indanazoline, metizoline; naphazoline, e.g. naphazoline hydrochloride; fenoxazoline, e. g. fenoxazoline hydrochloride; oxymetazoline, e.g. oxymetazoline hydrochloride; tetrahydrozoline, tramazoline, tyma-zoline; phenylephrine, e.g. phenylephrine hydrochloride; ephedrine, e.g. d-pseudoephedrine hydrochloride; or epine-phrine; or antiallergic agents, such as (1) cromoglycic acid ( = cromolyn) or a nasally acceptable salt thereof, e.g. the disodium salt ( = disodium cromoglycate), or (2) H1 receptor antagonists, e.g. dimethindene or a nasally acceptable salt thereof, e.g. dimethindene maleate; acrivastine, brompheniramine, chlorpheniramine, dexchlorpheniramine, tripro-lidine, bromodiphenhydramine, clemastine, phenyltoloxamine, piprinhydrinate, pyrilamine, tripelennamine, cetirizine, hydroxyzine, methdilazine, promethazine, trimeprazine, azatadine, cyproheptadine, loratadine, astemizole, diphenhy-dramine, levocabastine or terfenadine. Examples for corticosteroids are e.g. beclomethasone, e.g. beclomethasone dipropionate, or fluticasone, e.g. fluticasone propionate. Mixtures of more than one active substance come also into consideration, e.g. a combination of a vasoconstrictor and an antiallergic agent, such as xylometazoline plus cromoglycic acid or phenylephrine plus dimethindene, or a combination of a vasoconstrictor and a corticosteroid, such as xylometazoline plus beclomethasone.

Preferably, the active ingredient is xylometazoline or a pharmaceutically acceptable salt thereof. Xylometazoline is widely used in particular for treating symptoms of common cold and flu, where the skin irritation and redness issues arise frequently.

According to a preferred embodiment, the balm comprises an active ingredient having skin soothing properties, in particular selected from the group comprising ectoin, dexpanthenol, aloe vera, calendula, bisabolol and niacinamide.

Alternatively or in addition to such an active ingredient, the balm can contain skin conditioning or moisturing agents.

Preferably, the solidified balm has substantially a hemispherical shape, thus allowing for easy application.

### Brief description of the drawings

Fig. 1 shows a perspective view of a combination product according to a first embodiment of the invention
Fig. 2 shows a perspective view of the combination product of Fig. 1, with a base element of a balm holder element and a balm being taken off
Fig. 3 shows a base element and a balm according to the first embodiment of the invention
Fig. 4 shows a base element and a balm according to a second embodiment of the invention
Fig. 5 shows a perspective view of a combination product according to a third embodiment of the invention
Fig. 6 shows a perspective view of the combination product of Fig. 5, with a base element of a balm holder element and a balm being taken off
Fig. 7 shows an exploded view of the combination product of Fig. 1

### Detailed description of the invention

The invention will now be described by way of example only with reference to the accompanying figures.

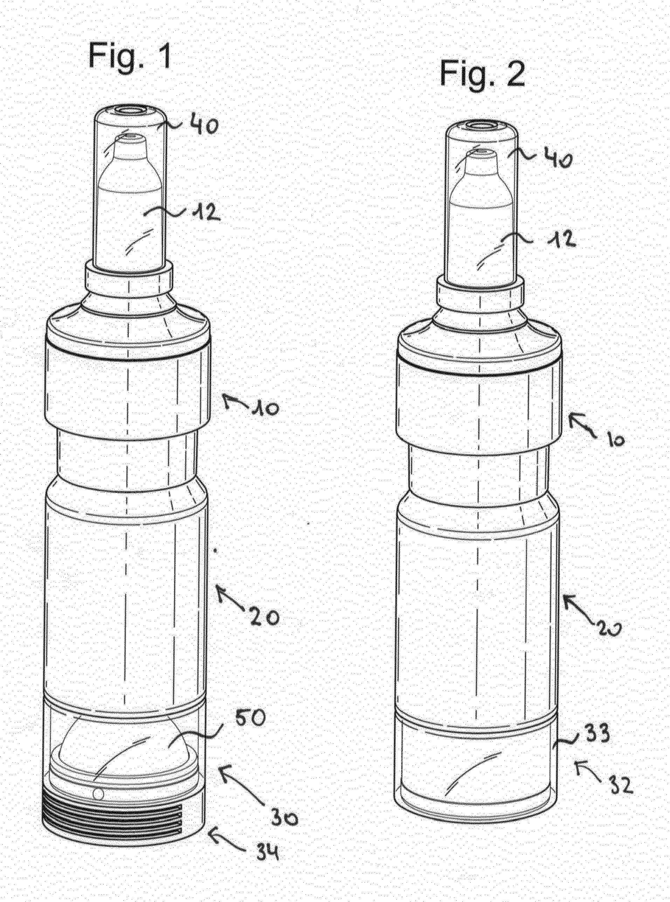

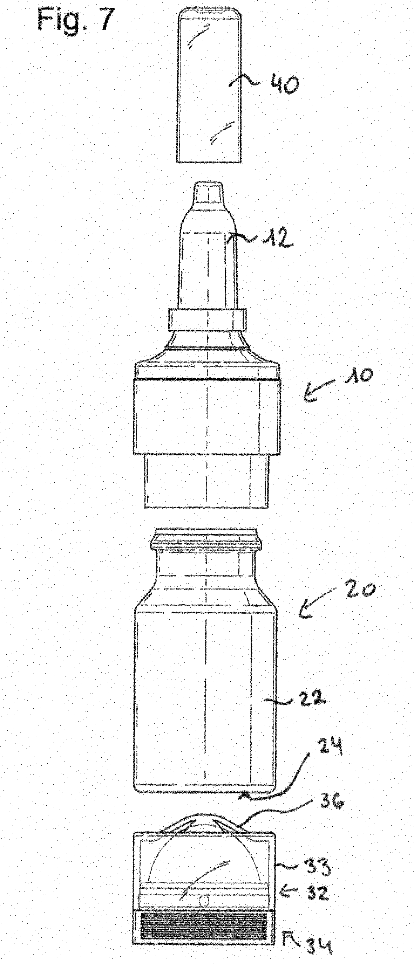

Figs. 1 and 7 shows a combination product which comprises a conventional nasal spray device comprising a HDPE container 20 containing a liquid formulation for nasal administration, and a dispensing head 10, here a metered dose spray pump, with a nozzle 12 to be introduced into a nostril, as well as a balm holder element 30 containing a solidified skin-care balm 50. The balm holder element 30 comprises a balm cover 32 and a base element 34 holding the balm 50.

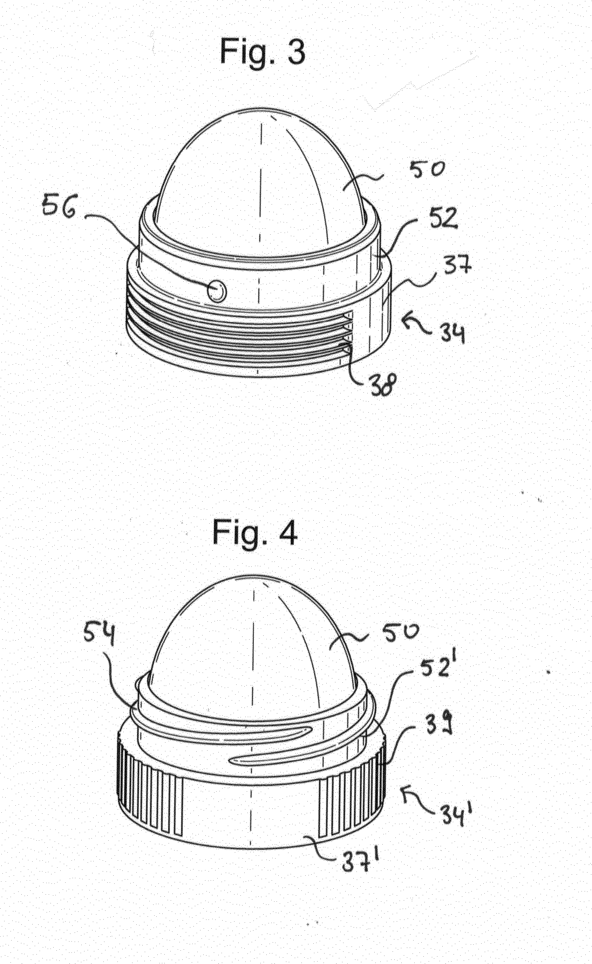

Fig. 2 shows the same combination product, where the base element 34 carrying the balm 50 has been taken off. Fig. 3 shows the base element 34 of the embodiment of Fig. 1, together with balm, separately and ready to be used. The base element 34 is moulded in one piece and has a circular base plate 37, with a circumferential rim 52 on that base plate 37 surrounding the solidified balm 50. Protrusions protruding inwardly from the circumferential rim 52 can be used to hold the solidified balm 50 in place, once it has been pressed on the base plate 37 and on same protrusions. In the first embodiment shown in Fig. 1 and 3, the base element 34 is provided with circumferential ribs 38 running along parts of the circumference of the base plate 37 and making it easier to grip the base plate 37 and thus the base element 34 with the balm 50.

The balm 50 has a substantially hemispherical shape, and an example for a formulation is given further below.

The balm cover 32 consists of a cylindrical portion 33 surrounding the balm 50, and a cover portion 36 which has a domed shape, as it can be seen in Fig. 7. The cover portion 36 with its domed shape matches a corresponding recess in the bottom 24 of the container 20, and it is glued on the bottom portion using a primer and cyano-acrylate glue. Both the balm cover 32 and the base element 34 are made of Polypropylene (PP).

On the outer surface of the rim 52, several protrusions 56 are foreseen, which make a snap-fit connection with the cylindrical portion 33 of the balm cover 32 and help to keep the base element 34 connected to the balm cover 32, and thus to the container 20.

Fig. 4 shows an alternative embodiment of a base element 34' carrying the same solidified balm 50. While the overall shape of the base plate 37' and the circumferential rim 52' are the same as for the first embodiment shown in Figs. 1-3, the rim 52' is in this embodiment provided with a thread 54 which matches a thread at the inner circumference of the cylindrical portion of the balm cover (not shown in the figures). Furthermore, in the embodiment shown in Fig. 4, the base element 34' also has ribs 39 on the outer circumference of the circular base plate 37', but these are perpendicular to the circumference of the base plate 37'.

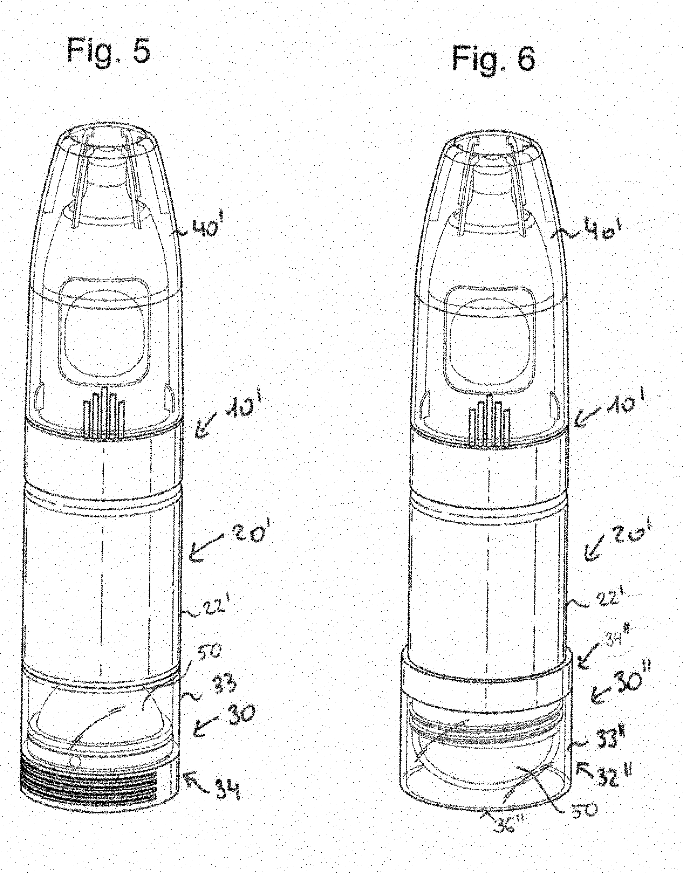

Fig. 5 shows an embodiment of a combination product according to the invention, differing from the embodiment shown in Fig. 1 in the choice of the container 20' and the metered dose spray pump 10'. Here, a laterally actuated pump 10' as for example described in WO2017191205A1 is used. The balm holder element 30 is the same as in the embodiment shown in Figs. 1-3.

Fig. 6 shows another embodiment of a combination product according to the invention, using the same container 20' and the same laterally actuated metered dose spray pump 10', but a different balm holder element 30". As it can be seen when comparing Figs. 5 and 6, the orientiation of the balm holder element 30" is inverted in the embodiment of Fig. 6, with the base element 34" being attached to the bottom of the container 20', and the balm cover 32" being removable. In this embodiment, the base element 34" is not glued on a base surface at the bottom of the container, but it comprises a cylindrical portion extending around the lowest part of the cylindrical wall 22' of the container 20', and can either be glued thereto, or fixed in another way, e.g. via a snap-fit or clip connection. The cover portion 36" of the balm cover 32" is flat, so that the combination product can stand on it.

An example for a balm formulation is given below in Table 1.

### Table 1: Example of a balm formulation

The Names of the raw materials are given using the International Nomenclature of Cosmetic Ingredients (INCI). The % are indicated by weight.

| **INCI name of component (ANALYTICAL)** | **% of component in the final formulation (analytical)** | **Function of the component** |
|---|---|---|
| OCTYLDODECANOL | 16.80 | EMOLLIENT |
| CAPRYLIC/CAPRIC TRIGLYCERIDE | 14.70 | SKIN CONDITIONING |
| RICINUS COMMUNIS SEED OIL | 14.30 | SKIN CONDITIONING |
| ORYZA SATIVA CERA | 14.00 | SKIN CONDITIONING |
| | | SKIN PROTECTING |
| | | SMOOTHING |
| RHUS VERNICIFLUA PEEL CERA/ RHUS SUCCEDANEA FRUIT CERA | 13.40 | EMOLLIENT |
| | | STABILISING / BINDING |
| | | EMOLLIENT |
| THEOBROMA CACAO SEED BUTTER | 7.00 | EMOLLIENT |
| | | SKIN CONDITIONING |
| PERSEA GRATISSIMA OIL | 5.00 | SKIN CONDITIONING |
| CETEARYL ALCOHOL | 3.00 | EMOLLIENT |
| | | EMULSIFYING |
| | | EMULSION STABILISING |
| MYRISTYL MYRISTATE | 3.00 | EMOLLIENT |
| | | SKIN CONDITIONING |
| POLYGLYCERYL-3 DIISOSTEARATE | 2.50 | EMULSIFYING |
| CETYL ESTERS | 2.00 | EMOLLIENT |
| | | SKIN CONDITIONING |
| BUTYROSPERMUM PARKII BUTTER | 2.00 | SKIN CONDITIONING |
| OLEYL ALCOHOL | 0.48 | EMOLLIENT |
| | | MASKING |
| ZANTHOXYLUM BUNGEANUM FRUIT EXTRACT | 0.12 | SKIN CONDITIONING |
| TOCOPHERYL ACETATE | 0.50 | ANTIOXIDANT |
| MENTHYL ETHYLAMIDO OXALATE | 0.50 | SKIN CONDITIONING |
| Eucalyptol Nat | 0.2 | PERFUMING |
| | | SKIN CONDITIONING |
| MENTHOL | 0.20 | REFRESHING |
| | | SOOTHING |
| BHT | 0.10 | ANTIOXIDANT |

## Claims

1. A combination product comprising a dispensing head (10, 10') mounted on a top of a container (20, 20') containing a liquid formulation for nasal administration, and a solidified skin-care balm (50) for application around the nose contained in a balm holder element (30, 30") attached to a bottom (24) of the container (20).

2. A combination product according to claim 1, wherein the dispensing head (10, 10') is a metered dose spray pump.

3. A combination product according to any of the preceding claims, wherein the balm holder element (30, 30") comprises a base element (34, 34', 34") on which the balm (50) is mounted, and a balm cover (32, 32") with a cylindrical portion (33, 33") surrounding the balm (50), and a cover portion (36, 36").

4. A combination product according to any of the preceding claims, wherein the cylindrical portion (33, 33") of the balm cover (32, 32") is aligned with a cylindrical wall (22, 22') of the container (20, 20') containing the liquid formulation.

5. A combination product according to any of the preceding claims, wherein the balm cover (32) is attached to the bottom (24) of the container.

6. A combination product according to claim 4, wherein the balm cover (32) is glued to the bottom (24) of the container (20, 20').

7. A combination product according to claim 4 or 5, wherein the cover portion (36) is dome-shaped and matches a recess in the bottom (24) of the container (20) containing the liquid formulation.

8. A combination product according to claim 4, wherein the balm cover is clipped or snap-fitted to the bottom (24) of the container.

9. A combination product according to any of claims 1 to 3, wherein the base element (34") is attached to the bottom (24) of the container (20').

10. A combination product according to claim 1, wherein the liquid formulation contains a saline solution and an active ingredient selected from the group of vasoconstrictors, antiallergic agents and corticosteroids.

11. A combination product according to claim 10, wherein the active ingredient is xylometazoline or a pharmaceutically acceptable salt thereof.

12. A combination product according to any of the preceding claims, wherein the balm comprises an active ingredient having skin soothing properties, in particular selected from the group comprising ectoin, dexpanthenol, aloe vera, calendula, bisabolol and niacinamide.

13. A combination product according to any of the preceding claims, wherein the solidified balm (50) has substantially a hemispherical shape.

14. A combination product according to any of the preceding claims, wherein the balm comprises one or several skin conditioning agents.

## Patentansprüche

1. Kombinationsprodukt, umfassend einen auf einer Oberseite eines Behälters (20, 20'), der eine flüssige Formulierung für die nasale Verabreichung enthält, montierten Spenderkopf (10, 10')und einen in einem Balsam-Halteelement (30, 30"), das mit einer Unterseite (24) des Behälters verbunden ist, enthaltenen verfestigten Hauptpflegebalsam (50) für die Auftragung um die Nase.

2. Kombinationsprodukt gemäß Anspruch 1, worin der Spenderkopf (10, 10') eine Einzeldosis-sprühpumpe ist.

3. Kombinationsprodukt gemäß irgendeinem der vorstehenden Ansprüche, worin das Balsam-Halteelement (30, 30") ein Basiselement (34, 34', 34"), auf dem der Balsam (50) angebracht ist, und eine Balsam-Abdeckung (32, 32") mit einem den Balsam (50) umgebenden zylindrischen Bereich (33, 33") und einem Abdeckbereich (36, 36") umfasst.

4. Kombinationsprodukt gemäß irgendeinem der vorstehenden Ansprüche, worin der zylindrische Bereich (33, 33") der Balsam-Abdeckung (32, 32") mit einer zylindrischen Wand (22, 22') des Behälters (20, 20'), der die flüssige Formulierung enthält, ausgerichtet ist.

5. Kombinationsprodukt gemäß irgendeinem der vorstehenden Ansprüche, worin die Balsam-Abdeckung (32) mit der Unterseite (24) des Behälters verbunden ist.

6. Kombinationsprodukt gemäß Anspruch 4, worin die Balsam-Abdeckung (32) mit der Unterseite (24) des Behälters (20, 20') verklebt ist.

7. Kombinationsprodukt gemäß Anspruch 4 oder 5, worin der Abdeckbereich (36) kuppelförmig ist und in eine Vertiefung in der Unterseite (24) des Behälters (20), der die flüssige Formulierung enthält, passt.

8. Kombinationsprodukt gemäß Anspruch 4, worin die Balsam-Abdeckung mit der Unterseite (24) des Behälters verklipst oder schnappverschlossen ist.

9. Kombinationsprodukt gemäß irgendeinem der Ansprüche 1 bis 3, worin das Basiselement (34") mit der Unterseite (24) des Behälters (20') verbunden ist.

10. Kombinationsprodukt gemäß Anspruch 1, worin die flüssige Formulierung eine Kochsalzlösung und einen Wirkstoff enthält, ausgewählt aus der Gruppe bestehend aus Vasokonstriktoren, Antiallergika und Corticosteroiden.

11. Kombinationsprodukt gemäß Anspruch 10, worin der Wirkstoff Xylometazolin oder ein pharmazeutisch akzeptables Salz hiervon ist.

12. Kombinationsprodukt gemäß irgendeinem der vorgehenden Ansprüche, worin der Balsam einen Wirkstoff mit hautberuhigenden Eigenschaften umfasst, insbesondere ausgewählt aus der Gruppe bestehend aus Ectoin, Dexpanthenol, Aloe Vera, Calendula, Bisbolol und Niacinamid.

13. Kombinationsprodukt gemäß irgendeinem der vorgehenden Ansprüche, worin der verfestigte Balsam (50) eine im Wesentlichen halbkugelförmige Form aufweist.

14. Kombinationsprodukt gemäß irgendeinem der vorgehenden Ansprüche, worin der Balsam ein oder mehrere Hautpflegemittel umfasst.

## Revendications

1. Association médicamenteuse comprenant une tête de distribution (10, 10') montée sur un sommet d'un récipient (20, 20') contenant une formulation liquide pour une administration nasale, et un baume (50) de soin de la peau solidifié pour une application autour du nez contenu dans un élément d'étui de baume (30, 30") fixé à un fond (24) du récipient (20).

2. Association médicamenteuse selon la revendication 1, dans laquelle la tête de distribution (10, 10') est une pompe de pulvérisation à dose mesurée.

3. Association médicamenteuse selon l'une quelconque des revendications précédentes, dans laquelle l'élément d'étui de baume (30, 30") comprend un élément de base (34, 34', 34") sur lequel le baume (50) est monté, et un couvercle de baume (32, 32") avec une partie cylindrique (33, 33") entourant le baume (50), et une partie de couverture (36, 36").

4. Association médicamenteuse selon l'une quelconque des revendications précédentes, dans laquelle la partie cylindrique (33, 33") du couvercle de baume (32, 32") est alignée avec une paroi cylindrique (22, 22') du récipient (20, 20') contenant la formulation liquide.

5. Association médicamenteuse selon l'une quelconque des revendications précédentes, dans laquelle le couvercle de baume (32) est fixé au fond (24) du récipient.

6. Association médicamenteuse selon la revendication 4, dans laquelle le couvercle de baume (32) est collé au fond (24) du récipient (20, 20').

7. Association médicamenteuse selon la revendication 4 ou 5, dans laquelle la partie de couverture (36) est en forme de dôme et s'adapte à un évidement dans le fond (24) du récipient (20) contenant la formulation liquide.

8. Association médicamenteuse selon la revendication 4, dans laquelle le couvercle de baume est clipsé ou encliqueté sur le fond (24) du récipient.

9. Association médicamenteuse selon l'une quelconque des revendications 1 à 3, dans laquelle l'élément de base (34") est fixé au fond (24) du récipient (20').

10. Association médicamenteuse selon la revendication 1, dans laquelle la formulation liquide contient une solution saline et un ingrédient actif choisi dans le groupe des vasoconstricteurs, des agents antiallergiques et des corticostéroïdes.

11. Association médicamenteuse selon la revendication 10, dans laquelle l'ingrédient actif est la xylométazoline ou un sel pharmaceutiquement acceptable de celle-ci.

12. Association médicamenteuse selon l'une quelconque des revendications précédentes, dans laquelle le baume comprend un ingrédient actif ayant des propriétés apaisantes pour la peau, notamment choisi dans le groupe comprenant l'ectoïne, le dexpanthénol, l'aloe vera, le calendula, le bisabolol et la niacinamide.

13. Association médicamenteuse selon l'une quelconque des revendications précédentes, dans laquelle le baume (50) solidifié présente une forme sensiblement hémisphérique.

14. Association médicamenteuse selon l'une quelconque des revendications précédentes, dans laquelle le baume comprend un ou plusieurs agents de conditionnement de la peau.
